(19) Europäisches Patentamt
European Patent Office
Office européen des brevets

(11) Numéro de publication : **0 308 283 B1**

# (12) FASCICULE DE BREVET EUROPEEN

(45) Date de publication du fascicule du brevet : **22.01.92 Bulletin 92/04**

(51) Int. Cl.$^5$ : **C07D 211/70,** A61K 31/445

(21) Numéro de dépôt : **88402128.8**

(22) Date de dépôt : **19.08.88**

## (54) Dérivés de l'oxime du 1,2,5,6-tétrahydropyridin-3-carboxaldéhyde, leur procédé de préparation, leur application comme médicaments et les compositions les renfermant.

(30) Priorité : **21.08.87 IT 2168787**

(43) Date de publication de la demande : **22.03.89 Bulletin 89/12**

(45) Mention de la délivrance du brevet : **22.01.92 Bulletin 92/04**

(84) Etats contractants désignés : **AT BE CH DE ES FR GB GR IT LI LU NL SE**

(56) Documents cités :
**FR-A- 1 258 847**
**CHEMICAL ABSTRACTS, vol. 92, no. 13, 31 mars 1980, page 641, résumé no. 110798y, Columbus, Ohio, US; I.A. KOZELLO et al.: "Improvement in the method of preparing arecoline based on acetaldehyde"**

(73) Titulaire : **ROUSSEL-UCLAF**
**35, Boulevard des Invalides**
**F-75007 Paris (FR)**

(72) Inventeur : **Galliani, Giulio**
**Via Silva 13**
**I-20052 Monza (MI) (IT)**
Inventeur : **Barzaghi, Fernando**
**Via Monteverdi 21**
**I-20052 Monza (MI) (IT)**
Inventeur : **Bonetti, Carla**
**Via Beccalino**
**I-Fontanella (BG) (IT)**
Inventeur : **Toja, Emilio**
**Via Plezzo 80**
**I-20100 Milano (IT)**

(74) Mandataire : **Tonnellier, Marie-José et al**
**111, route de Noisy B.P. no 9**
**F-93230 Romainville (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux dérivés de l'oxime du 1,2,5,6,-tétrahydropyridin-3-carboxaldéhyde, leur procédé de préparation et leur application comme médicaments.

L'invention a pour objet les composés de formule (I) :

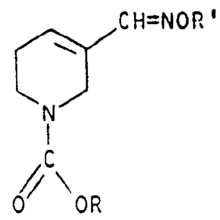

(I)

dans laquelle R′ représente un atome d'hydrogène, un radical alkyle linéaire, ramifié ou cyclique, saturé ou insaturé renfermant jusqu'à 8 atomes de carbone, et R représente un radical alkyle linéaire, ramifié ou cyclique, saturé ou insaturé renfermant jusqu'à 18 atomes de carbone éventuellement substitué par le radical alkylsulfonyle renfermant jusqu'à 18 atomes de carbone ou le radical Si-$Alk_3$ dans lequel Alk représente un radical alkyle renfermant jusqu'à 4 atomes de carbone un radical phényle, benzyle ou phénéthyle éventuellement substitué par un ou plusieurs substituants choisis parmi les atomes d'halogène, les radicaux méthyle, éthyle, propyle linéaire ou ramifié, butyle linéaire ou ramifié, les radicaux méthoxy, éthoxy, propoxy linéaire ou ramifié et butoxy linéaire ou ramifié, ainsi que leurs sels d'addition avec les acides organiques ou minéraux.

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, propionique, benzoïque, maléïque, fumarique, succinique, tartrique, citrique, oxalique, glyoxylique, aspartique, alcanesulfoniques tels que les acides méthane ou éthane sulfoniques, arylsulfoniques tels que les acides benzène ou paratoluènesulfoniques.

Lorsque R représente un radical saturé, linéaire ou ramifié, il s'agit de préférence d'un radical méthyle, éthyle, n-propyle, isopropyle, n-butyle, isobutyle, n-pentyle, n-hexyle, tert-butyle, néopentyle, ou adamantyle.

Lorsque R′ représente un radical alkyle saturé, linéaire ou ramifié, il s'agit de préférence d'un radical méthyle, éthyle, n-propyle, isopropyle, n-butyle, isobutyle, n-pentyle, tert-butyle, tert-pentyle, néopentyle ou n-hexyle.

Lorsque R ou R′ représente un radical alkyle insaturé, il s'agit de préférence d'un radical éthylénique comme, par exemple, le radical vinyle, allyle, 1,1-diméthylallyle, but-2-ényle, ou d'un radical acétylénique comme, par exemple, le radical éthynyle ou propynyle.

Lorsque R ou R′ représente un radical alkyle cyclique, il s'agit de préférence d'un radical cyclopropyle, cyclobutyle, cyclopentyle, cyclohexyle, cyclopropylméthyle, cyclobutylméthyle, cyclopentylméthyle ou cyclohexylméthyle.

Lorsque le radical R représente un radical phényle, benzyle ou phénéthyle substitué par un ou plusieurs atomes d'halogène, il s'agit de préférence d'un ou plusieurs atomes de chlore ou de brome.

Lorsque R représente un radical alkyle substitué par un alkylsulfonyle, il s'agit de préférence du radical $-CH_2SO_2CH_3$, $-CH_2CH_2SO_2CH_3$ ou $-CH_2CH_2CH_2SO_2CH_3$.

Parmi les composés préférés de l'invention, on peut citer les composés, dans lesquels R′ représente un radical alkyle linéaire, saturé ou insaturé renfermant jusqu'à 4 atomes de carbone, par exemple un radical méthyle.

On peut citer également les composés dans lesquels R représente un radical alkyle linéaire, saturé ou insaturé renfermant jusqu'à 4 atomes de carbone comme par exemple un radical méthyle ou encore un radical allyle ou un radical phényle, un radical phényle substitué par l'un des substituants indiqués précédemment, comme par exemple un radical 4-chlorophényle, isopropylphényle ou 3,4-diméthoxyphényle ainsi que leurs sels d'addition avec les acides minéraux ou organiques.

Comme composés préférés de l'invention, on peut citer les composés décrits aux exemples 2, 4, 6, 13 et 14 et plus particulièrement l'éther méthylique de l'oxime du 4-chlorophényloxycarbonyl 1,2,5,6-tétrahydropyridin-3-carboxaldéhyde ainsi que leurs sels d'addition avec les acides organiques ou minéraux.

Les produits de l'invention présentent de très intéressantes propriétés pharmacologiques notamment une activité cholinomimétique par voie orale importante et de longue durée d'action.

Les produits présentent de plus une forte dissociation entre l'activité centrale et l'activité périphérique comme le montrent les résultats des tests exposés ci-après. Il est bien connu que les troubles d'apprentissage

et de la mémoire chez les personnes âgées sont surtout reliés à un déficit du système cholinergique central, en particulier dans la démence sénile et la maladie d'Alzheimer.

Il est donc évident que les produits ayant une action cholinergique centrale puissent être employés dans le traitement thérapeutique de ces maladies (Bartus, R.I. Science 217, 408, 1982).

Il a été démontré que l'arécoline injectée par voie intraveineuse a un effet positif sur des patients ayant un déficit de la mémoire (Sitaram N. et al. Science 201, 274, 1978) (Christie J.E. et al. Brit. J. Psychiatry 138, 46, 1981).

Une limitation à l'emploi thérapeutique de l'arécoline est liée au fait que ce produit a une très faible activité par voie orale et une courte durée d'action.

Les produits, objet de l'invention, ont montré, après administration par voie orale, une activité cholinomimétique centrale très supérieure à celle de l'arécoline et avec une plus longue durée d'action.

L'invention a donc pour objet les produits de l'invention en tant que médicaments utiles notamment dans le traitement de la maladie d'Alzheimer ou de la démence sénile et également dans le traitement des troubles de la mémoire.

L'invention a plus particulièrement pour objet en tant que médicaments, les composés des exemples 2, 4, 6, 12, 13 et 14 ainsi que leurs sels d'addition avec les acides pharmaceutiquement acceptables.

La posologie usuelle est variable selon l'affection en cause, le sujet traité et la voie d'administration ; elle peut être comprise entre 10 mg et 300 mg/jour, par exemple, entre 15 et 150 mg/jour en une ou plusieurs prises pour le produit de l'exemple 12 administré par voie orale.

La présente invention a également pour objet les compositions pharmaceutiques renfermant comme principe actif au moins un produit de formule (I).

Les compositions pharmaceutiques selon l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation, caractérisé en ce que l'on soumet un composé de formule (II) :

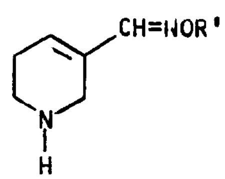

(II)

dans laquelle R' conserve la même signification que précédemment à l'action d'un agent d'alcoxycarbonylation capable d'introduire le radical —$CO_2R$, R étant défini comme précédemment pour obtenir le composé de formule (I) correspondant, que l'on soumet si désiré à l'action d'un acide pour en former le sel.

Dans un mode de réalisation préférée du procédé de l'invention, l'agent d'alcoxycarbonylation est un produit de formule (III) :

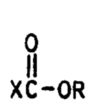

(III)

dans laquelle X représente un atome d'halogène, un radical paranitrophénoxy ou un radical

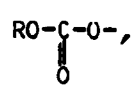

R étant défini comme précédemment.

L'invention a également pour objet un procédé caractérisé en ce que l'on soumet un composé de formule (IV) :

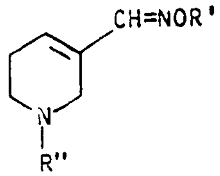

(IV)

dans laquelle R″ représente un radical alkyle linéaire, ramifié ou cyclique, saturé ou insaturé, renfermant jusqu'à 4 atomes de carbone ou un radical benzyle ou phénéthyle renfermant jusqu'à 14 atomes de carbone, à l'action d'un agent capable de cliver le groupement R″ et d'introduire le groupement

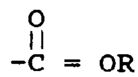

R étant défini comme précédemment pour obtenir le composé de formule (I) correspondant :

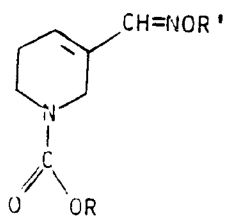

(I)

que l'on soumet si désiré à l'action d'un acide pour en former le sel.

Dans un mode de réalisation préféré, on utilise un composé de formule (III) :

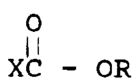

(III)

en opérant à chaud.

Les produits de départ de formule (II) et (IV) sont décrits et revendiqués dans la demande de brevet européen EP 0239445.

Les exemples suivants illustrent l'invention sans toutefois la limiter.

**Exemple 1 : Ether méthylique de l'oxime de la 1-méthoxycarbonyle 1,2,5,6-tétrahydropyridin-3-carboxaldéhyde.**

A une solution de 1,5 g de 1,2,5,6-tétrahydropyridin-3-carboxaldéhyde-O-méthyl oxime préparé comme indiqué dans la demande de brevet EP 239445 dans 40 cm³ de benzène, on ajoute à 10°C 1,5 cm³ de triéthylamine et, lentement, 0,83 cm³ de chloroformiate de méthyle. On agite 30 minutes a température ambiante, lave avec 15 cm³ d'acide chlorhydrique à 5% puis à l'eau. On évapore à sec sous pression réduite puis distille le résidu à 140°C sous 0,05 mbar. On obtient 1,91 g de produit attendu. F = 37°C.

| | | | | |
|---|---|---|---|---|
| Analyse | : | $C_9H_{14}N_2O_3$ | | |
| Calculé | : | C % 54,33 | H % 7,12 | N % 14,13 |
| Trouvé | : | 54,31 | 7,17 | 14,00 |

**Exemple 2 : Ether méthylique de l'oxime de la 1-éthoxycarbonyle 1,2,5,6-tétrahydropyridin-3-carboxaldéhyde.**

Méthode A :

A une solution de 3,6 g de 1-benzyl 1,2,5,6-tétrahydropyridin-3-carboxaldéhyde-O-méthyloxime préparé comme indiqué dans la demande de brevet EP 239445 dans 30 cm³ de dichloroéthane, on ajoute 2 cm³ de chloroformiate d'éthyle et maintient 3 heures au reflux. On refroidit le mélange réactionnel et lave la phase organique avec 20 cm³ d'acide chlorhydrique à 5% puis avec 2 fois 25 cm³ d'eau. On évapore le solvant et distille l'huile résiduelle à 150°C sous 0,05 mbar. On obtient 1,5 g de produit attendu.

| Analyse : | $C_{10}H_{16}N_2O_3$ | | |
|---|---|---|---|
| Calculé : | C% 56,59 | H% 7,60 | N% 13,20 |
| Trouvé : | 56,82 | 7,66 | 13,04 |

Méthode B :

A une solution de 3,75 g de 1,2,5,6-tétrahydropyridin-3-carboxaldéhyde-O-méthyloxime préparé comme indiqué dans la demande de brevet EP 239445 dans 60 cm³ de benzène, on ajoute à une température ne dépassant pas 10°C, 3,75 cm³ de triéthylamine et 2,7 cm³ de chloroformiate d'éthyle. On agite 1 heure à température ambiante, ajoute 20 cm³ d'eau, décante et extrait avec du benzène. On évapore à sec sous pression réduite et chromatographie le résidu sur silice (éluant : acétate d'éthyle-toluène 1-1). On distille le résidu à 160-165°C sous 0,07 mbar. On obtient 5,32 g de produit attendu.

| Analyse : | $C_{10}H_{16}N_2O_3$ | | |
|---|---|---|---|
| Calculé : | C% 56,59 | H% 7,60 | N% 13,20 |
| Trouvé : | 56,41 | 7,54 | 13,31 |

**Exemple 3 : Ether méthylique de l'oxime de la 1-n-propyloxycarbonyle 1,2,5,6-tétrahydropyridin-3-carboxaldéhyde.**

A une solution de 1,5 g de 1,2,5,6-tétrahydropyridin-3-carboxaldéhyde-O-méthyloxime dans 60 cm³ de benzène, on ajoute 1,5 cm³ de triéthylamine et en maintenant la température entre 5 et 10°C, on ajoute lentement 1,23 cm³ de chloroformiate de propyle. Après 30 minutes à température ambiante, on lave avec 20 cm³ d'acide chlorhydrique 1N puis à l'eau. On évapore le solvant sous pression réduite et distille à 170°C sous 0,07 mbar. On obtient 2,3 g de produit attendu.

| Analyse : | $C_{11}H_8N_2O_3$ | | |
|---|---|---|---|
| Calculé : | C% 58,39 | H% 8,02 | N% 12,38 |
| Trouvé : | 58,12 | 8,06 | 12,12 |

**Exemple 4 : Ether méthylique de l'oxime de la 1-allyloxycarbonyle 1,2,5,6-tétrahydropyridin-3-carboxaldéhyde.**

A une solution de 1,2,5,6-tétrahydropyridin-3-carboxaldéhyde-O-méthyl oxime dans 40 cm³ de benzène, on ajoute à 5°C 1,5 cm³ de triéthylamine et 1,17 cm³ de chloroformiate de 2-propényle. On agite pendant 30 minutes à température ambiante puis traite avec 20 cm³ d'acide chlorhydrique à 5% puis avec 2 fois 20 cm³ d'eau. On évapore le solvant puis distille le résidu à 200°C sous 0,08 mbar. On obtient 2,25 g de produit attendu.

| | | | |
|---|---|---|---|
| Analyse : | $C_{11}H_{16}N_2O_3$ | | |
| Calculé : | C% 58,91 | H% 7,19 | N% 12,49 |
| Trouvé : | 58,96 | 7,25 | 12,41 |

**Exemple 5 : Ether méthylique de l'oxime de la 1-benzyloxycarbonyle 1,2,5,6-tétrahydropyridin-3-carboxaldéhyde.**

Méthode A :

A une solution de 8 g de 1-benzyl 1,2,5,6-tétrahydropyridin-3-carboxaldéhyde-O-méthyloxime dans 100 $cm^3$ de benzène, on ajoute 23 $cm^3$ de chloroformiate de benzyle à 50% dans le toluène. On porte 4 heures à l'ébullition, refroidit, lave à l'acide chlorhydrique 2N puis à l'eau. On évapore le solvant sous pression réduite puis distille à 200°C sous 0,05 mbar. On obtient 4,1 g de produit attendu.

Méthode B :

A une solution de 1,5 g de 1,2,5,6-tétrahydropyridin-3-carboxaldéhyde-O-méthyloxime dans 20 $cm^3$ de benzène et 1,08 g de triéthylamine, on ajoute lentement à 10°C 3,4 $cm^3$ de chloroformiate de benzyle à 50% dans le toluène. On agite 1 heure à température ambiante, lave avec de l'acide chlorhydrique à 5% dans l'eau, évapore le solvant et distille le résidu à 230°C sous 0,06 mbar. On obtient 2,1 g de produit attendu.

| | | | |
|---|---|---|---|
| Analyse : | $C_{14}H_{16}N_2O_3$ | | |
| Calculé : | C% 65,54 | H% 6,72 | N% 10,14 |
| Trouvé : | 65,68 | 6,61 | 10,21 |

**Exemple 6 : Ether méthylique de l'oxime de la 1-phényloxycarbonyle 1,2,5,6-tétrahydropyridin-3-carboxaldéhyde.**

Méthode A :

A une solution de 3,3 g de 1-méthyl 1,2,5,6-tétrahydropyridin-3-carboxaldéhyde-O-méthyloxime préparé comme indiqué dans la demande de brevet EP239445 dans 30 $cm^3$ de dichloréthane, on ajoute 4,5 g de chloroformiate de phényle et chauffe pendant 3 heures au reflux. On filtre l'insoluble (chlorhydrate du produit de départ) puis évapore à sec sous pression réduite, on chromatographie le résidu sur silice (éluant : benzène-acétate d'éthyle 8-2). On obtient 4 g de produit attendu après distillation à 220°C sous 0,06 mbar.

Méthode B :

A une solution de 3 g de 1-éthyl 1,2,5,6-tétrahydropyridin-3-carboxaldéhyde-O-méthyloxime préparé comme indiqué dans la demande de brevet EP 239445 dans 30 $cm^3$ de dichloréthane, on ajoute 4,7 $cm^3$ de chloroformiate de phényle. On porte au reflux pendant 3 heures puis traite avec 20 $cm^3$ d'acide chlorhydrique à 5% puis 2 fois avec 20 $cm^3$ d'eau. On évapore le solvant puis reprend à l'éther diéthylique, filtre l'insoluble et évapore à sec le filtrat. On distille l'huile résiduelle à 210°C sous 0,03 mbar. On obtient 2,18 g de produit attendu.

| | | | |
|---|---|---|---|
| Analyse : | $C_{14}H_{16}N_2O_3$ | | |
| Calculé : | C% 64,6 | H% 6,20 | N% 10,76 |
| Trouvé : | 64,72 | 6,35 | 10,65 |

**Exemple 7 : Ether méthylique de l'oxime de la [1-(2-méthylsulfonyl) éthoxycarbonyle] 1,2,5,6-tétrahydropyridin-3-carboxaldéhyde.**

A une solution de 1,5 g de 1,2,5,6-tétrahydropyridin-3-carboxaldéhyde-O-méthyloxime dans 40 cm³ de benzène anhydre et 1,5 cm³ de triéthylamine que l'on refroidit à 5°C, on ajoute 3,1 g de carbonate de (2-méthylsulfonyl) éthyl 4-nitrophényle. On poursuit la réaction 1 heure à température ambiante, lave à l'eau la phase organique, puis évapore à sec sous pression réduite. On chromatographie le résidu sur silice (éluant : acétate d'éthyle) et obtient 1,6 g de produit attendu cristallisé du benzène. F = 93-94°C.

| Analyse : | $C_{11}H_{18}N_2O_5S$ | | |
|---|---|---|---|
| Calculé : | C% 45,50 | H% 6,25 | N% 9,65 |
| Trouvé : | 45,63 | 6,29 | 9,61 |

**Exemple 8 : Ether méthylique de l'oxime de la 1-adamantyloxycarbonyle 1,2,5,6-tétrahydropyridin-3-carboxaldéhyde.**

A une solution de 1,5 g de 1,2,5,6-tétrahydropyridin-3-carboxaldéhyde-O-méthyloxime dans 40 cm³ de benzène et 1,5 cm³ de triéthylamine, on ajoute à 5°C 2,13 g de fluoroformiate d'adamantyle. Après 15 minutes à température ambiante, on lave avec de l'acide chlorhydrique 2N puis à l'eau. On évapore à sec sous pression réduite et cristallise de l'hexane. On obtient 2,6 g de produit attendu. F = 105-106°C.

| Analyse : | $C_{18}H_{26}N_2O_3$ | | |
|---|---|---|---|
| Calculé : | C% 67,90 | H% 8,23 | N% 8,80 |
| Trouvé : | 68,02 | 8,28 | 8,76 |

**Exemple 9 : Ether méthylique de l'oxime de la 1-isopropyloxycarbonyle 1,2,5,6-tétrahydropyridin-3-carboxaldéhyde.**

A un mélange de 1,4 g de 1,2,5,6-tétrahydropyridin-3-carboxaldéhyde-O-méthyloxime et 1,01 g de triéthylamine anhydre dans 30 cm³ de benzène, on ajoute à 10°C 1,22 g de chloroformiate d'isopropyle en solution benzénique. On agite 30 minutes à température ambiante, décante, lave la phase organique, évapore à sec et chromatographie le résidu sur silice (éluant : toluène-acétate d'éthyle 8-2). On distille le résidu à 180°C sous 0,08 mbar. On obtient 1,35 g de produit attendu. F = 40-41°C.

| Analyse : | $C_{11}H_{18}N_2O_3$ | | |
|---|---|---|---|
| Calculé : | C% 58,39 | H% 8,02 | N% 12,38 |
| Trouvé : | 58,19 | 8,13 | 12,54 |

**Exemple 10 : Ether méthylique de l'oxime de la 1-butyloxycarbonyle 1,2,5,6-tétrahydropyridin-3-carboxaldéhyde.**

A une solution de 1,5 g de 1,2,5,6-tétrahydropyridin-3-carboxaldéhyde-O-méthyloxime dans 20 cm³ de benzène et 1,2 g de triéthylamine, on ajoute à 10°C 1,46 g de chloroformiate de butyle. On agite 2 heures à température ambiante, lave avec de l'acide chlorhydrique 2N puis évapore à sec et distille le résidu à 220°C sous 0,08 mbar. On obtient 1.85 g de produit attendu.

| Analyse : | $C_{12}H_{20}N_2O_3$ | | |
|---|---|---|---|
| Calculé : | C% 59,98 | H% 8,39 | N% 11,66 |
| Trouvé : | 59,72 | 8,49 | 11,77 |

**Exemple 11 : Ether méthylique de l'oxime de la 1-[2-(phényl) éthyloxy] carbonyle 1,2,5,6-tétrahydropyridin-3-carboxaldéhyde.**

A un mélange de 1,4 g de 1,2,5,6-tétrahydropyridin-3-carboxaldéhyde-O-méthyloxime et 1,01 g de triéthylamine dans 40 cm$^3$ de benzène, on ajoute à + 10°C 1,85 g de chloroformiate de [(2-phényl) éthyle]. Après 30 minutes à température ambiante, on lave avec de l'acide chlorhydrique dilué et évapore à sec. On purifie par chromatographie sur alumine (éluant : cyclohexane-acétate d'éthyle 9-1) puis distille le résidu sous 0,07 mbar à 250°C. On obtient 2,2 g de produit attendu.

| | | | |
|---|---|---|---|
| Analyse : | $C_{16}H_{20}N_2O_3$ | | |
| Calculé : | C% 66,65 | H% 6,99 | N% 9,72 |
| Trouvé : | 66,51 | 7,10 | 9,79 |

**Exemple 12 : Ether méthylique de l'oxime de la 1-parachlorophényloxycarbonyle 1,2,5,6-tétrahydropyridin-3-carboxaldéhyde.**

A un mélange de 1,4 g de 1,2,5,6-tétrahydropyridin-3-carboxaldéhyde-O-méthyloxime et 1,01 g de triéthylamine dans 40 cm$^3$ de benzène, on ajoute à 10°C 1,91 g de chloroformiate de p-chlorophényle (J. Am. Chem. Soc. 47, 2607) dans 30 cm$^3$ de benzène. Après 3 heures à température ambiante, on lave à l'acide chlorhydrique dilué et évapore à sec. On purifie par chromatographie sur alumine (éluant : cyclohexane-acétate d'éthyle 7-3). On obtient 1,9 g de produit attendu cristallisé du cyclohexane. F = 96-98°C

| | | | |
|---|---|---|---|
| Analyse : | $C_{14}H_{15}N_2O_3$ | | |
| Calculé : | C% 57,05 | H% 5,13 | N% 9,51 |
| Trouvé : | 56,78 | 4,97 | 9,37 |

**Exemple 13 : Ether méthylique de l'oxime de la 1-p-isopropylphényloxy carbonyle 1,2,5,6-tétrahydropyridin-3-carboxaldéhyde.**

A 1,4 g de 1,2,5,6-tétrahydropyridin-3-carboxaldéhyde-O-méthyloxime dans 40 cm$^3$ de benzène et 1,01 g de triéthylamine, on ajoute à 10°C 1,99 g de chloroformiate de p-isopropylphényle (British Patent 798659 (1978)) en 5 solution dans 20 cm$^3$ de benzène. Après 1 nuit à température ambiante, on lave avec de l'acide chlorhydrique dilué puis évapore à sec. On purifie par chromatographie sur alumine (éluant : cyclohexane-acétate d'éthyle 9-1). On obtient après cristallisation dans le cyclohexane 2 g de produit attendu. F = 100-102°C.

| | | | |
|---|---|---|---|
| Analyse : | $C_{17}H_{22}N_2O_3$ | | |
| Calculé : | C% 67,53 | H% 7,33 | N% 9,27 |
| Trouvé : | 67,70 | 7,44 | 9,14 |

**Exemple 14 : Ether méthylique de l'oxime de la 1-(3,4-diméthoxyphényloxy carbonyle 1,2,5,6-tétrahydropyridin-3-carboxaldéhyde.**

A une solution de 1,2 g de 1,2,5,6-tétrahydropyridin-3-carboxaldéhyde-O-méthyloxime dans 20 cm$^3$ de benzène 1,2 cm$^3$ de triéthylamine, on ajoute 1,86 g de chloroformiate de 3,4-diméthoxyphényle. On agite pendant 45 minutes et lave à l'acide chlorhydrique à 10% et évapore à sec. On chromatographie le résidu sur alumine (éluant : cyclohexane-acétate d'éthyle 7-3). On obtient 1,85 g de produit attendu cristallisé du cyclohexane. F = 143-145°C,

| | | | |
|---|---|---|---|
| Analyse : | $C_{16}H_{20}N_2O_5$ | | |
| Calculé : | C% 59,99 | H% 6,29 | N% 8,75 |
| Trouvé : | 60,24 | 6,21 | 8,61 |

**Exemple 15 : Ether 2-propynylique de l'oxime de la 1-éthyloxycarbonyle 1,2,5,6-tétrahydropyridin-3-carboxaldéhyde.**

A une solution de 1,2,5,6-tétrahydropyridin-3-carboxaldéhyde-O-(2-propynyloxime préparé comme indiqué dans la demande de brevet EP 239445 dans 50 cm³ de benzène, on ajoute à 5°C 1,42 cm³ de triéthylamine et 1,02 cm³ de chloroformiate d'éthyle à 95%. On agite 30 minutes puis lave à l'eau et évapore à sec sous pression réduite. On purifie par chromatographie sur alumine (éluant : benzène-éther diéthylique 1-1). On évapore à sec et reprend avec du benzène, traite avec du sulfate de sodium et du charbon actif, filtre et amène à sec sous pression réduite. On obtient 1,95 g de produit attendu. F = 55°C.

| | | | |
|---|---|---|---|
| Analyse : | $C_{12}H_{16}N_2O_3$ | | |
| Calculé : | C% 61,00 | H% 6,83 | N% 11,86 |
| Trouvé : | 61,23 | 6,66 | 11,71 |

**Exemple 16 : Ether méthylique de l'oxime de la 1-terbutyloxycarbonyle 1,2,5,6-tétrahydropyridin-3-carboxaldéhyde.**

A une solution de 1,5 g de 1,2,5,6-tétrahydropyridin-3-carboxaldéhyde-O-méthyloxime dans 40 cm³ de benzène et 1,5 g de triéthylamine. On refroidit à 5°C et on ajoute 2,33 g de carbonate de terbutyle. Après 10 minutes à température ambiante, on lave avec de l'acide chlorhydrique 2N puis à l'eau. On évapore sous pression réduite puis isole le produit par distillation à 150°C sous 0,07 mbar. On obtient 2,2 g de produit attendu.

| | | | |
|---|---|---|---|
| Analyse : | $C_{12}H_{20}N_2O_3$ | | |
| Calculé : | C% 59,83 | H% 8,5 | N% 11,49 |
| Trouvé : | 60,00 | 8,39 | 11,66 |

**Exemple 17 : Ether méthylique de l'oxime de la 1-(2-triméthylsilyléthyloxy carbonyle 1,2,5,6-tétrahydropyridin-3-carboxaldéhyde.**

A une solution de 1,5 g de 1,2,5,6,-tétrahydropyridin-3-carboxaldéhyde-O-méthyloxime dans 40 cm³ de benzène et 1,5 cm³ de triéthylamine, on ajoute à 5°C 3,03 g de paranitrophényl carbonate de (2-triméthylsilyl) éthyle. On agite 3 heures à température ambiante, on lave à l'eau puis évapore sous vide. On purifie par chromatographie sur silice (éluant : benzène-éther diéthylique 1-1) et isole le produit attendu par distillation à 220°C sous 0,08 mbar. On obtient 2,3 g de produit attendu.

| | | | |
|---|---|---|---|
| Analyse : | $C_{13}H_{24}N_2O_3Si$ | | |
| Calculé : | C% 54,89 | H% 8,50 | N% 9,85 |
| Trouvé : | 54,85 | 8,59 | 9,76 |

**Exemple de compositions pharmaceutiques.**

a) On a préparé des comprimés répondant à la formule suivante :

- Produit de l'exemple 12 .................................... 50 mg
- Excipient q.s. pour un comprimé terminé à .................... 300 mg

(Détail de l'excipient : lactose, amidon de blé, amidon traité, amidon de riz, stéarate de magnésium, talc).
b) On a préparé des gélules répondant à la formule suivante :

- Produit de l'exemple 12 .................................... 60 mg
- Excipient q.s. pour un gélule terminée à .................... 300 mg

(Détail de l'excipient : talc, stéarate de magnésium, Aérosil®)

## ETUDE PHARMACOLOGIQUE

### Toxicité aigüe.

L'essai est réalisé sur des souris mâles ($CD_1$ Charles Rivers) de 22 à 24 g, à jeun depuis 16 heures. On administre les produits par voie orale à la dose de 1000, 500, 250, 125 et 62 mg/kg. On note la mortalité pendant les 7 jours suivant le traitement.

| Exemple | $DL_{50}$ mg/kg |
|---|---|
| 2 | 175 |
| 4 | 175 |
| 6 | > 500 |
| 13 | > 1000 |
| 12 | > 1000 |
| 14 | 175 |
| Arécoline HBr | 600 |

### Test de l'iléon isolé de cobaye.

On prélève des fragments d'iléon de cobayes tués par décapitation. L'iléon isolé est placé dans 10 $cm^3$ de solution de Tyrode à 37°C et aéré par un mélange d'oxygène (95%) et de gaz carbonique (5%). Les contractions dues aux produits sont enregistrées à l'aide d'un capteur relié à un polygraphe. Les produits à tester sont ajoutés aux concentrations comprises entre $1.10^{-3}$M et $1.10^{-8}$M/l.

Les produits présentant un effet contracturant sont testés vis à vis de l'atropine et de l'hexaméthonium pour établir si l'activité est de type "muscarinique" ou "nicotinique".

L'activité antagoniste éventuelle des produits est testés vis à vis de l'acétylcholine.

L'activité agoniste est exprimée en $pD_2$ (logarithme négatif de la dose qui produit 50% de l'effet maximum).

L'activité antagoniste est exprimée en $DE_{50}$ (dose réduisant de 50% la réponse maximale induite par l'acétylcholine).

| Exemple | $pD_2$ | $DE_{50}$ mg/kg |
|---|---|---|
| 2 | 4,47 | - |
| 4 | < 4 | 6,0 x $10^{-4}$ |
| 6 | < 3 | > 1 x $10^{-3}$ |
| 13 | < 4 | > 1 x $10^{-4}$ |
| 12 | < 4 | > 1 x $10^{-4}$ |
| 14 | < 4 | > 1 x $10^{-4}$ |
| Arécoline | 6,48 | - |

**Activité diarrhéique.**

Le test est réalisé sur des souris mâles ($CD_1$ Charles Rivers) pesant 25 à 30 g, à jeun depuis 6 heures. Le produit dissous à 5% dans du méthocel est administré par voie orale, au moyen d'une sonde oesophagienne.

Des animaux témoins ne reçoivent que l'excipient.

Après traitement, les animaux sont mis séparément dans des cages dont le fond est recouvert de papier buvard et sont mis en observation pendant 30, 60, 120 et 180 minutes.

Les feuilles de papier absorbant sont changées après chaque observation.

La consistance des fèces est évaluée selon la méthode de Randall et Baruth (Arch. Int. Pharmacodyn. 220, 94, 1976) en suivant l'échelle des valeurs suivantes.

0 : consistance ferme,
1 : fèces légèrement molles avec ou sans auréole humide,
2 : fèces légèrement molles avec présence d'un cercle humide bien défini,
3 : fèces molles avec présence d'un grand cercle humide,
4 : fèces sans consistance avec présence d'un très grand cercle humide.

Pour chaque produit, on a noté la dose qui provoque une diarrhée chez 50% des animaux selon la méthode de Miller et Tainter (Proc. Soc. Exp. Biol. Med., 57, 261, 1944).

| Exemple | $DE_{50}$ mg/kg |
|---|---|
| 2 | 25 |
| 4 | 3 |
| 6 | 100 |
| 13 | > 50 |
| 12 | > 50 |
| 14 | > 50 |
| Arécoline | 35 |

**Activité hypothermique.**

Le test est réalisé sur des souris mâles ($CD_1$ Charles Rivers) pesant 25 à 30 g, à jeun depuis 6 heures.

La température du corps est notée au moyen d'un thermocouple placé dans le rectum à environ 1,5 cm, et relié à un enregistreur de température électrique.

Les produits sont administrés par voie orale ou sous-cutanée et les températures sont notées à l'instant 0 et 30 minutes, 1 heure, 2 heures et 2 heures et demie après traitement.

On évalue le degré d'hypothermie comme la différence entre les animaux traités et les témoins et on détermine la dose nécessaire pour réduire de 1°C la température du corps.

| Exemple | Dose efficace (-1°C) en mg/kg | |
|---|---|---|
| | V.O | V.SC |
| 2 | 3,9 | 3,6 |
| 4 | 1,6 | 1,8 |
| 6 | 4,6 | 2,3 |
| 13 | 3,4 | 8,8 |
| 12 | 2,2 | 2,9 |
| 14 | 0,7 | 0,7 |
| Arécoline | 194 | 3,0 |

**Variation de la température corporelle.**

On détermine la durée d'action des produits en utilisant des doses capables de réduire la température de 1° à 1,5°C.

Variations de la température du corps (°C)

| Exemple | dose mg/kg | administration | temps en minutes de traitement 0 | 30 | 60 | 120 | 180 |
|---|---|---|---|---|---|---|---|
| 2 | 5 | os | + 0,2 | - 1,2** | - 1,1** | - 0,1 | ± 0 |
| | 5 | sc | + 0,2 | - 1,5** | - 1,4** | - 0,1 | ± 0 |
| 4 | 2 | os | - 0,1 | - 1,2** | - 1,1** | - 0,1 | - 0,1 |
| | 2 | sc | - 0,1 | - 0,9** | - 1,0** | - 0,2 | - 0,1 |
| 6 | 7,5 | os | - 0,1 | - 1,1** | - 1,6** | - 0,6** | ± 0 |
| | 3,5 | sc | - 0,1 | - 0,6** | - 1,4** | - 0,9** | + 0,1 |
| 13 | 6,25 | os | + 0,2 | - 0,4** | - 1,6** | - 0,4** | - 0,1 |
| | 12,5 | sc | + 0,2 | ± 0 | - 0,7** | - 1,4** | - 1,1** |
| 12 | 3,12 | os | + 0,1 | - 0,9** | - 1,4** | - 0,3* | ± 0 |
| | 3,12 | sc | - 0,1 | - 0,1 | - 1,1** | - 1,2** | - 0,5** |
| 14 | 1 | os | + 0,1 | - 1,1** | - 1,4** | - 0,2 | + 0,1 |
| | 1 | sc | + 0,2 | - 0,7** | - 1,3** | - 0,5** | ± 0 |
| Arécoline,HBr | 200 | os | + 0,1 | - 1,1** | - 1,0** | - 0,2 | - 0,1 |
| | 3,5 | sc | - 0,1 | - 1,5** | - 0,1 | + 0,2 | + 0,2 |

** Valeurs différentes des témoins d'une manière significative ($p < 0,05$); (* $p < 0,01$)

## Revendications

### Revendications pour les Etats contractants suivants : AT, BE, CH, DE, FR, GB, IT, LI, LU, NL, SE

1. Les composés de formule (I) :

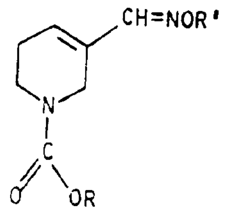

(I)

dans laquelle R′ représente un atome d'hydrogène, un radical alkyle linéaire, ramifié ou cyclique, saturé ou insaturé renfermant jusqu'à 8 atomes de carbone et R représente un radical alkyle linéaire, ramifié ou cyclique, saturé ou insaturé renfermant jusqu'à 18 atomes de carbone éventuellement substitué par le radical alkylsulfonyle renfermant jusqu'à 18 atomes de carbone ou le radical $Si-Alk_3$ dans lequel Alk représente un radical alkyle renfermant jusqu'à 4 atomes de carbone, un radical phényle, benzyle ou phénéthyle éventuellement substitué, par un ou plusieurs substituants choisis parmi les atomes d'halogène, les radicaux méthyle, éthyle, propyle linéaire ou ramifié, butyle linéaire ou ramifié, les radicaux méthoxy, éthoxy, propoxy linéaire ou ramifié et butoxy linéaire ou ramifié, ainsi que leurs sels d'addition avec les acides organiques ou minéraux.

2. Les composés de formule (I), tels que définis à la revendication 1, dans lesquels R′ représente un radical alkyle linéaire, saturé ou insaturé renfermant jusqu'à 4 atome de carbone, ainsi que leurs sels d'addition avec les acides organiques ou minéraux.

3. Les composés de formule (I), tels que définis à la revendication 2, dans lesquels R′ représente un radical méthyle, ainsi que leurs sels d'addition avec les acides organiques ou minéraux.

4. Les composés de formule (I), tels que définis à l'une quelconque des revendications 1 à 3, dans lesquels R représente un radical alkyle linéaire, saturé ou insaturé renfermant jusqu'à 4 atomes de carbone ainsi que leurs d'addition avec les acides organiques ou minéraux.

5. Les composés de formule (I), tels que définis à la revendication 4, dans lesquels R représente un radical éthyle, ainsi que leurs sels d'addition avec les acides organiques et minéraux.

6. Les composés de formule (I), tels que définis à la revendication 4, dans lesquels R représente un radical allyle, ainsi que leurs sels d'addition avec les acides organiques ou minéraux.

7. Les composés de formule (I), tels que définis à l'une quelconque des revendications 1 à 3, dans lesquels R représente un radical phényle ainsi que leurs sels d'addition avec les acides organiques ou minéraux.

8. Les composés de formule (I), tels que définis à l'une quelconque des revendications 1 à 3, dans lesquels R représente un radical phényle substitué par un ou plusieurs des radicaux mentionnés ci-dessus, ainsi que leurs sels d'addition avec les acides organiques ou minéraux.

9. Les composés de formule (I), tels que définis à l'une quelconque des revendications 1 à 3, dans lesquels R représente un radical 4-chloro phényle, ainsi que leurs sels d'addition avec les acides organiques ou minéraux.

10. Les composés de formule (I), tels que définis à l'une quelconque des revendications 1 à 3, dans lesquels R représente un radical 4-isopropyl phényle, ainsi que leurs sels d'addition avec les acides organiques ou minéraux.

11. Les composés de formule (I), tels que définis à l'une quelconque des revendications 1 à 3, dans lesquels R représente un radical 3,4-diméthoxy phényle ainsi que leurs sels d'addition avec les acides organiques ou minéraux.

12. Le composé de formule (I) tel que défini à la revendication 1 dont le nom suit :

— l'éther méthylique de l'oxime du 4-chlorophényloxycarbonyl 1,2,5,6-tétrahydropyridin-3-carboxaldéhyde ainsi que ses sels d'addition avec les acides organiques ou minéraux.

13. A titre de médicaments, les composés de formule (I) tels que définis à l'une quelconque des revendications 1 à 11, ainsi que leurs sels d'addition avec les acides organiques ou minéraux pharmaceutiquement acceptables.

14. A titre de médicament, le composé de formule (I) tel que défini à la revendication 12, ainsi que ses sels d'addition avec les acides pharmaceutiquement acceptables.

15. Les compositions pharmaceutiques renfermant comme principe actif au moins l'un des médicaments définis à la revendication 13 ou 14.

16. Procédé de préparation des composés de formule (I), tels que définis à l'une quelconque des revendications 1 à 12, caractérisé en ce que l'on soumet un composé de formule (II) :

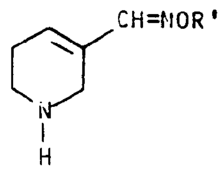

(II)

dans laquelle R′ conserve la même signification que précédemment, à l'action d'un agent d'alcoxycarbonylation capable d'introduire le radical —$CO_2R$, R étant défini comme précédemment, pour obtenir le composé de formule (I) correspondant, que l'on soumet si désiré à l'action d'un acide pour former le sel.

17. Procédé de préparation selon la revendication 16, caractérisé en ce que l'agent d'alcoxycarbonylation est un produit de formule (III) :

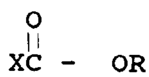

(III)

dans laquelle X représente un atome d'halogène, un radical paranitrophénoxy ou un radical

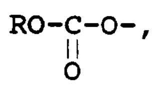

R étant défini comme précédemment.

18. Variante du procédé selon la revendication 16 ou 17, caractérisé en ce l'on soumet un procédé de formule (IV) :

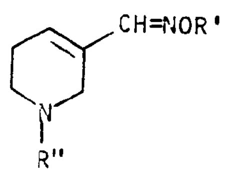

(IV)

dans laquelle R″ représente un radical alkyle linéaire, ramifié ou cyclique, saturé ou insaturé renfermant jusqu'à 4 atomes de carbone ou un radical benzyle ou phénéthyle, à l'action d'un agent capable de cliver le groupement R″ et d'introduire le groupement

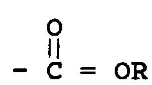

R étant défini comme précédemment pour obtenir le composé de formule (I) correspondant :

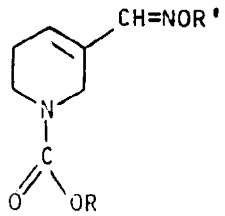

(I)

que l'on soumet si désiré à l'action d'un acide pour en former le sel.

**Revendications pour les Etats contractants suivants : ES, GR**

1. Procédé pour préparer les composés de formule (I) :

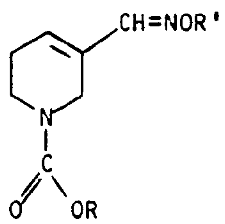

(I)

dans laquelle R′ représente un atome d'hydrogène, un radical alkyle linéaire, ramifié ou cyclique, saturé ou insaturé renfermant jusqu'à 8 atomes de carbone, et R représente un radical alkyle linéaire, ramifié ou cyclique, saturé ou insaturé renfermant jusqu'à 18 atomes de carbone éventuellement substitué par le radical alkylsulfonyle renfermant jusqu'à 18 atomes de carbone ou le radical Si-$Alk_3$ dans lequel Alk représente un radical alkyle renfermant jusqu'à 4 atomes de carbone, un radical phényle, benzyle ou phénéthyle éventuellement substitué, par un ou plusieurs substituants choisis parmi les atomes d'halogène, les radicaux méthyle, éthyle, propyle, linéaire ou ramifié, butyle linéaire ou ramifié, les radicaux méthoxy, éthoxy, propoxy linéaire ou ramifié et butoxy linéaire ou ramifié, ainsi que leurs sels d'addition avec les acides organiques ou minéraux, caractérisé en ce que l'on soumet un composé de formule (II) :

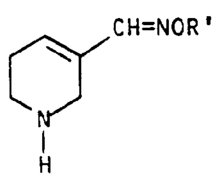

(II)

dans laquelle R′ conserve la même signification que précédemment, à l'action d'un agent d'alcoxycarbonylation capable d'introduire le radical —$CO_2R$, R étant défini comme précédemment, pour obtenir le composé de formule (I) correspondant, que l'on soumet si désiré à l'action d'un acide pour former le sel.

2. Procédé de préparation selon la revendication 1, caractérisé en ce que l'agent d'alcoxycarbonylation est un produit de formule (III) :

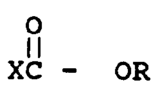

(III)

dans laquelle X représente un atome d'halogène, un radical paranitrophénoxy ou un radical

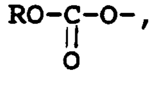

R étant défini comme précédemment.

3. Variante du procédé selon la revendication 1 ou 2, caractérisé en ce l'on soumet un procédé de formule (IV) :

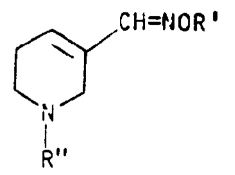

(IV)

dans laquelle R″ représente un radical alkyle linéaire, ramifie ou cyclique, sature ou insaturé, renfermant jusqu'a 4 atomes de carbone ou un radical benzyle ou phénéthyle, à l'action d'un agent capable de cliver le groupement R″ et d'introduire le groupement

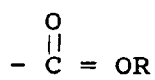

R étant défini comme précédemment pour obtenir le composé de formule (I) correspondant :

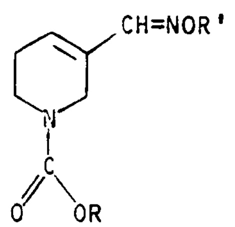

(I)

que l'on soumet si désiré à l'action d'un acide pour en former le sel.

4. Procédé selon la revendication 1, 2 ou 3 caractérisé en ce que l'on utilise au départ un produit capable d'introduire le radical —$CO_2R$ dans lequel R représente un radical alkyle linéaire ou ramifié ou cyclique, saturé ou insaturé renfermant jusqu'à 18 atomes de carbone, un radical phényle, un radical benzyle ou phénéthyle.

5. Procédé selon l'une quelconque des revendications 1 à 3 caractérisé en ce que l'on utilise au départ un produit de formule (II) ou (IV) dans laquelle R′ représente un radical alkyle linéaire, saturé ou insaturé renfermant jusqu'à 4 atomes de carbone ainsi que leurs sels d'addition avec les acides organiques ou minéraux.

6. Procédé selon l'une quelconque des revendication 1 à 3 caractérisé en ce que l'on utilise au départ un produit de formule (II) ou (IV) dans laquelle R′ représente un radical méthyle, ainsi que leurs sels d'addition avec les acides organiques ou minéraux.

7. Procédé selon la revendication 4, caractérisé en ce que l'on utilise au départ un produit de formule (II) ou (IV) dans laquelle R′ représente un radical méthyle ainsi que leurs sels d'addition avec les acides organiques ou minéraux.

8. Procédé selon la revendication 4, caractérisé en ce que l'on utilise au départ un produit capable d'introduire le radical —$CO_2R$ dans lequel R représente un radical alkyle linéaire, saturé ou insaturé, renfermant jusqu'à 4 atomes de carbone ainsi que leurs sels d'addition avec les acides organiques ou minéraux.

9. Procédé selon la revendication 4, caractérisé en ce que l'on utilise au départ un produit capable d'introduire le radical —$CO_2R$ dans lequel R représente un radical éthyle, ainsi que leurs sels d'addition avec les acides organiques ou minéraux.

10. Procédé selon la revendication 4, caractérisé en ce que l'on utilise au départ un produit capable d'introduire le radical —$CO_2R$ dans lequel R représente un radical allyle, ainsi que leurs sels d'addition avec les acides organiques ou minéraux.

11. Procédé selon la revendication 4, caractérisé en ce que l'on utilise au départ un produit capable d'introduire le radical —$CO_2R$ dans lequel R représente un radical phényle, ainsi que leurs sels d'addition avec les acides organiques ou minéraux.

12. Procédé selon l'une quelconque des revendications 1 à 3, caractérisé en ce que l'on utilise au départ un produit capable d'introduire le radical —$CO_2R$ dans lequel R représente un radical phényle substitué par un ou plusieurs des radicaux mentionnés ci-dessus, ainsi que leurs sels d'addition avec les acides organiques ou minéraux.

13. Procédé selon l'une quelconque des revendications 1 a 3, caractérisé en ce que l'on utilise au départ un produit capable d'introduire le radical —$CO_2R$ dans lequel R représente un radical 4-chloro phenyle, 4-isopropyl phenyle ou 3,4-diméthoxy phényle, ainsi que leurs sels d'addition avec les acides organiques ou minéraux.

14. Procédé selon la revendication 1 ou 3 pour la préparation de l'éther méthylique de l'oxime du 4-chlorophényloxycarbonyl 1,2,5,6-tétrahydropyridin-3-carboxaldéhyde ou l'un de ses sels d'addition avec les acides organiques ou minéraux, caractérisé en ce que l'on utilise au départ un produit de formule (II) ou (IV) dans laquelle R′ représente un radical méthyle et un produit capable d'introduire le radical —$CO_2R$ dans lequel R représente un radical 4-chloro phényle.

## Patentansprüche

### Patentansprüche für folgende Vertragsstaaten : AT, BE, CH, DE, FR, GB, IT, LI, LU, NL, SE

1. Verbindungen der Formel (I)

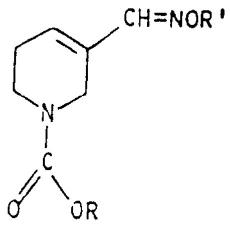

(I)

worin R′ für ein Wasserstoffatom, einen gesättigten oder ungesättigten, linearen, verzweigten oder cyclischen Alkylrest mit bis zu 8 Kohlenstoffatomen steht und R einen gesättigten oder ungesättigten, linearen, verzweigten oder cyclischen Alkylrest mit bis zu 18 Kohlenstoffatomen bedeutet, der gegebenenfalls substituiert ist durch den Alkylsulfonylrest mit bis zu 18 Kohlenstoffatomen oder den Si-$Alk_3$-Rest, worin Alk für einen Alkylrest mit bis zu 4 Kohlenstoffatomen steht, einen Phenyl-, Benzyl-oder Phenethylrest, der gegebenenfalls substituiert ist durch einen oder mehrere Substituenten, ausgewählt unter den Halogenatomen, den Methyl-, Ethyl-, linearen oder verzweigten Propyl-, linearen oder verzweigten Butylresten, den Methoxy-, Ethoxy-, linearen oder verzweigten Propoxy- und linearen oder verzweigten Butoxyresten, sowie deren Additionssalze mit organischen oder Mineralsäuren.

2. Die Verbindungen der Formel (I), wie in Anspruch 1 definiert, worin R′ für einen gesättigten oder ungesättigten, linearen Alkylrest mit bis zu 4 Kohlenstoffatomen steht, sowie deren Additionssalze mit organischen oder Mineralsäuren.

3. Die Verbindungen der Formel (I) gemäß Anspruch 2, worin R′ für einen Methylrest steht, sowie deren Additionssalze mit organischen oder Mineralsäuren.

4. Die Verbindungen der Formel (I) gemäß einem der Ansprüche 1 bis 3, worin R für einen gesättigten oder ungesättigten, linearen Alkylrest mit bis zu 4 Kohlenstoffatomen steht, sowie deren Additionssalze mit organischen oder Mineralsäuren.

5. Die Verbindungen der Formel (I) gemäß Anspruch 4, worin R für einen Ethylrest steht, sowie deren Additionssalze mit organischen und Mineralsäuren.

6. Die Verbindungen der Formel (I) gemäß Anspruch 4, worin R für einen Allylrest steht, sowie deren Additionssalze mit organischen oder Mineralsäuren.

7. Die Verbindungen der Formel (I) gemäß einem der Ansprüche 1 bis 3, worin R für einen Phenylrest steht, sowie deren Additionssalze mit organischen oder Mineralsäuren.

8. Die Verbindungen der Formel (I) gemäß einem der Ansprüche 1 bis 3, worin R für einen Phenylrest steht, der durch einen oder mehrere der vorstehend erwähnten Reste substituiert ist, sowie deren Additionssalze mit organischen oder Mineralsäuren.

9. Die Verbindungen der Formel (I) gemäß einem der Ansprüche 1 bis 3, worin R einen 4-Chlorphenylrest bedeutet, sowie deren Additionssalze mit organischen oder Mineralsäuren.

10. Die Verbindungen der Formel (I) gemäß einem der Ansprüche 1 bis 3, worin R für einen 4-Isopropylphenylrest steht, sowie deren Additionssalze mit organischen oder Mineralsäuren.

11. Die Verbindungen der Formel (I) gemäß einem der Ansprüche 1 bis 3, worin R für einen 3,4-Dimethoxyphenylrest steht, sowie deren Additionssalze mit organischen oder Mineralsäuren.

12. Die Verbindung der Formel (I) gemäß Anspruch 1 mit der folgenden Bezeichnung :
Methylether des Oxims von 4-Chlorphenyloxycarbonyl-1,2,5,6-tetrahydropyridin-3-carboxaldehyd sowie dessen Additionssalze mit organischen oder Mineralsäuren.

13. Als Arzneimittel die Verbindungen der Formel (I), wie in einem der Ansprüche 1 bis 11 definiert, sowie deren pharmazeutisch verträgliche Additionssalze mit organischen oder Mineralsäuren.

14. Als Arzneimittel die Verbindung der Formel (I), wie in Anspruch 12 definiert, sowie deren Additionssalze mit pharmazeutisch verträglichen Säuren.

15. Pharmazeutische Zusammensetzungen, enthaltend als Wirkstoff zumindest eines der Arzneimittel gemäß Anspruch 13 oder 14.

16. Verfahren zur Herstellung der Verbindungen der Formel (I) gemäß einem der Ansprüche 1 bis 12, dadurch gekennzeichnet, daß man eine Verbindung der Formel (II)

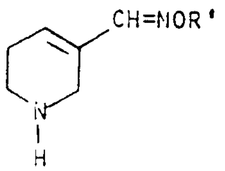

(II)

worin R′ die vorstehend angegebene Bedeutung besitzt, der Einwirkung eines Alkoxycarbonylierungsmittels unterzieht, das in der alge ist, den Rest —$CO_2R$, worin R wie vorstehend definiert ist, einzuführen, um zu der entsprechenden Verbindung der Formel (I) zu gelagen, die man gewünschtenfalls der Einwirkung einer Säure unterzieht, um das Salz zu bilden.

17. Verfahren gemäß Anspruch 16, dadurch gekennzeichnet, daß das Alkoxycarbonylierungsmittel ein Produkt der Formel (III)

$$X\overset{O}{\overset{\|}{C}} - OR \qquad (III)$$

ist, worin X ein Halogenatom, einen Paranitrophenoxyrest oder einen Rest

$$RO-\underset{O}{\underset{\|}{C}}-O-$$

bedeutet, worin R wie vorstehend definiert ist.

18. Abänderung des Verfahrens gemäß Anspruch 16 oder 17, dadurch gekennzeichnet, daß man eine Verbindung der Formel (IV)

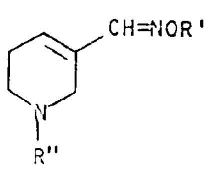

(IV)

worin R″ einen gesättigten oder ungesättigten, linearen, verzweigten oder cyclischen Alkylrest mit bis zu 4 Kohlenstoffatomen oder einen Benzyl- oder Phenethylrest bedeutet, der Einwirkung eines Mittels unterzieht, das befähigt ist, die Gruppe R" abzuspalten und die Gruppe

$$-\overset{O}{\overset{\|}{C}} = OR,$$

worin R wie vorstehend defiliert ist, einzuführen, um zu der entsprechenden Verbindung der Formel (I)

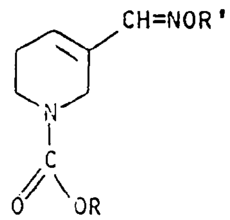

(I)

zu gelangen, die man gewünschtenfalls der Einwirkung einer Säure unterzieht, um hieraus das Salz zu bilden.

**Patentansprüche für folgende Vertragsstaaten : ES, GR**

1. Verfahren zur Herstellung der Verbindungen der Formel (I)

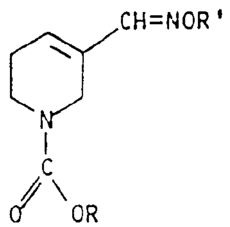

(I)

worin R′ für ein Wasserstoffatom, einen gesättigten oder ungesättigten, linearen, verzweigten oder cyclischen Alkylrest mit bis zu 8 Kohlenstoffatomen steht und R einen gesättigten oder ungesättigten, linearen, verzweigten oder cyclischen Alkylrest mit bis zu 18 Kohlenstoffatomen bedeutet, der gegebenenfalls substituiert ist durch den Alkylsulfonylrest mit bis zu 18 Kohlenstoffatomen oder den Si-$Alk_3$-Rest, worin Alk für einen Alkylrest mit bis zu 4 Kohlenstoffatomen steht, einen Phenyl-, Benzyl- oder Phenethylrest, der gegebenenfalls substituiert ist durch einen oder mehrere Substituenten, ausgewählt unter den Halogenatomen, den Methyl-, Ethyl-, linearen oder verzweigten Propyl-, linearen oder verzweigten Butylresten, den Methoxy-, Ethoxy-, linearen oder verzweigten Propoxy- und linearen oder verzweigten Butoxyresten, sowie von deren Additionssalzen mit organischen oder Mineralsäuren, dadurch gekennzeichnet, daß man eine Verbindung der Formel (II)

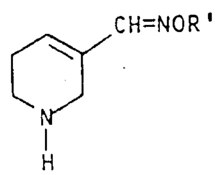

(II)

worin R′ die vorstehend angegebene Bedeutung besitzt, der Einwirkung eines Alkoxycarbonylierungsmittels unterzieht, das befähigt ist, den Rest —$CO_2R$, worin R wie vorstehend definiert ist, einzuführen, um zu der entsprechenden Verbindung der Formel (I) zu gelangen, die man gewünschtenfalls der Einwirkung einer Säure unterzieht, um das Salz zu bilden.

2. Verfahren gemäß Anspruch 1, dadurch gekennzeichnet, daß das Alkoxycarbonylierungsmittel ein Produkt der Formel (III)

$$X\overset{\overset{O}{\|}}{C} - OR \qquad (III)$$

ist, worin X für ein Halogenatom, einen Paranitrophenoxyrest oder einen Rest

$$RO-\underset{\underset{O}{\|}}{C}-O- ,$$

steht, in dem R wie vorstehend definiert ist.

3. Abänderung des Verfahrens gemäß Anspruch 1 oder 2, dadurch gekennzeichnet, daß man eine Verbindung der Formel (IV)

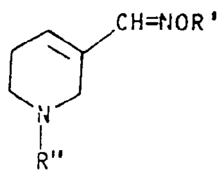

(IV)

worin R″ einen gesättigten oder ungesättigten, linearen, verzweigten oder cyclischen Alkylrest mit bis zu 4 Kohlenstoffatomen oder einen Benzyl- oder Phenethylrest bedeutet, der Einwirkung eines Mittels unterzieht, das befähigt ist, die Gruppe R″ abzuspalten und die Gruppe

$$-\overset{\overset{\displaystyle O}{\|}}{C} = OR,$$

worin R wie vorstehend definiert ist, einzuführen, um zu der entsprechenden Verbindung der Formel (I)

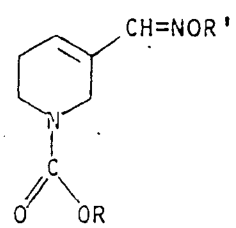

(I)

zu gelangen, die man gewünschtenfalls der Einwirkung einer Säure unterzieht, um hieraus das Salz zu bilden.

4. Verfahren gemäß Anspruch 1, 2 oder 3, dadurch gekennzeichnet, daß von einem Produkt ausgeht, das befähigt ist, den Rest —$CO_2R$, worin R einen gesättigten oder ungesättigten, linearen, verzweigten oder cyclischen Alkylrest mit bis zu 18 Kohlenstoffatomen, einen Phenylrest, einen Benzyl- oder einen Phenethylrest bedeutet, einzuführen.

5. Verfahren gemäß einem der Ansprüche 1 bis 3, dadurch gekennzeichnet, daß man von einem Produkt der Formel (II) oder (IV), worin R′ für einen gesättigten oder ungesättigten, linearen Alkylrest mit bis zu 4 Kohlenstoffatomen steht, sowie von dessen Additionssalzen mit organischen oder Mineralsäuren ausgeht.

6. Verfahren gemäß einem der Ansprüche 1 bis 3, dadurch gekennzeichnet, daß man von einem Produkt der Formel (II) oder (IV), worin R′ für einen Methylrest steht, sowie von dessen Additionssalzen mit organischen oder Mineralsäuren ausgeht.

7. Verfahren gemäß Anspruch 4, dadurch gekennzeichnet, daß man von einem Produkt der Formel (II) oder (IV), worin R′ für einen Methylrest steht, sowie von dessen Additionssalzen mit organischen oder Mineralsäuren ausgeht.

8. Verfahren gemäß Anspruch 4, dadurch gekennzeichnet, daß man von einem Produkt, das befähigt ist, den Rest —$CO_2R$ einzuführen, worin R für einen gesättigten oder ungesättigten, linearen Alkylrest mit bis zu 4 Kohlenstoffatomen steht, sowie von dessen Additionssalzen mit organischen oder Mineralsäuren ausgeht.

9. Verfahren gemäß Anspruch 4, dadurch gekennzeichnet, daß man von einem Produkt, das befähigt ist, den Rest —$CO_2R$ einzuführen, worin R für einen Ethylrest steht, sowie von dessen Additionssalzen mit organischen oder Mineralsäuren ausgeht.

10. Verfahren gemäß Anspruch 4, dadurch gekennzeichnet, daß man von einem Produkt, das befähigt ist, den Rest —$CO_2R$ einzuführen, worin R für einen Allylrest steht, sowie von dessen Additionssalzen mit organischen oder Mineralsäuren ausgeht.

11. Verfahren gemäß Anspruch 4, dadurch gekennzeichnet, daß man von einem Produkt, das befähigt ist, den Rest—$CO_2R$ einzuführen, worin R für einen Phenylrest steht, sowie von dessen Additionssalzen mit organischen oder Mineralsäuren ausgeht.

12. Verfahren gemäß den Ansprüchen 1 bis 3, dadurch gekennzeichnet, daß man von einem Produkt, das befähigt ist, den Rest —$CO_2R$ einzuführen, worin R für einen Phenylrest steht, der substituiert ist durch einen oder mehrere der vorstehend erwähnten Reste, sowie von dessen Additionssalzen mit organischen oder Mineralsäuren ausgeht.

13. Verfahren gemäß den Ansprüchen 1 bis 3, dadurch gekennzeichnet, daß man von einem Produkt, das befähigt ist, den Rest —$CO_2R$ einzuführen, worin R für einen 4-Chlorphenyl-, 4-Isopropylphenyl- oder 3,4-Dimethoxyphenylrest steht, sowie von dessen Additionssalzen mit organischen oder Mineralsäuren ausgeht.

14. Verfahren gemäß Anspruch 1 oder 3 zur Herstellung des Methylethers des Oxims von 4-Chlorphenyloxy-carbonyl-1,2,5,6-tetrahydropyridin-3-carboxaldehyd oder eines seiner Additionssalze mit organischen oder Mineralsäuren, dadurch gekennzeichnet, daß man von einem Produkt der Formel (II) oder (IV), worin R′ für einen Methylrest steht, und einem Produkt ausgeht, das in der Lage ist, den Rest—$CO_2R$ einzuführen, worin R für einen 4-Chlorphenylrest steht.

## Claims

### Claims for the following Contracting States : AT, BE, CH, DE, FR, GB, IT, LI, LU, NL, SE

1. The compounds of formula (I) :

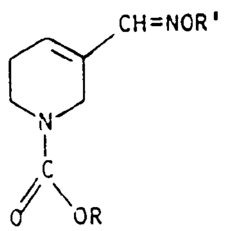

(I)

in which R′ represents a hydrogen atom, a saturated or unsaturated, linear, branched or cyclic alkyl radical containing up to 8 carbon atoms and R represents a saturated or unsaturated, linear, branched or cyclic alkyl radical containing up to 18 carbon atoms optionally substituted by the alkylsulphonyl radical containing up to 18 carbon atoms or the Si-$Alk_3$ radical in which Alk represents an alkyl radical containing up to 4 carbon atoms, a phenyl, benzyl or phenethyl radical optionally substituted by one or more substituents chosen from halogen atoms or the following radicals : methyl, ethyl, linear or branched propyl, linear or branched butyl, methoxy, ethoxy, linear or branched propoxy and linear or branched butoxy, as well as their addition salts with organic or mineral acids.

2. The compounds of formula (I), as defined in claim 1, in which R′ represents a saturated or unsaturated, linear alkyl radical containing up to 4 carbon atoms, as well as their addition salts with organic or mineral acids.

3. The compounds of formula (I), as defined in claim 2, in which R′ represents a methyl radical, as well as their addition salts with organic or mineral acids.

4. The compounds of formula (I) as defined in any one of claims 1 to 3, in which R represents a saturated or unsaturated, linear alkyl radical containing up to 4 carbon atoms as well as their addition salts with organic or mineral acids.

5. The compounds of formula (I), as defined in claim 4, in which R represents an ethyl radical, as well as their addition salts with organic or mineral acids.

6. The compounds of formula (I), as defined in claim 4, in which R represents an allyl radical as well as their addition salts with organic or mineral acids.

7. The compounds of formula (I), as defined in any one of claims 1 to 3, in which R represents a phenyl radical as well as their addition salts with organic or mineral acids.

8. The compounds of formula (I), as defined in any one of claims 1 to 3, in which R represents a phenyl radical, substituted by one or more of the radicals mentioned above, as well as their addition salts with organic or mineral acids.

9. The compounds of formula (I), as defined in any one of claims 1 to 3, in which R represents a 4-chloro phenyl radical, as well as their addition salts with organic or mineral acids.

10. The compounds of formula (I), as defined in any one of claims 1 to 3, in which R represents a 4-isopropyl phenyl radical, as well as their addition salts with organic or mineral acids.

11. The compounds of formula (I), as defined in any one of claims 1 to 3, in which R represents a 3,4-dimethoxy phenyl radical as well as their addition salts with organic or mineral acids.

12. The compound of formula (I) as defined in claim 1 the name of which follows :

— the methyl ether of 4-chlorophenyloxycarbonyl-1,2,5,6-tetrahydropyridin-3-carboxaldehyde oxime as well as its addition salts with organic or mineral acids.

13. As medicaments, the compounds of formula (I) as defined in any one of claims 1 to 11, as well as their addition salts with organic or mineral acids.

14. As a medicament, the compound of formula (I) as defined in claim 12, as well as its addition salts with organic or mineral acids.

15. The pharmaceutical compositions containing as active ingredient at least one of the medicaments defined in claim 13 or 14.

16. Preparation process for the compounds of formula (I), as defined in any one of claims 1 to 12, characterized in that a compound of formula (II) :

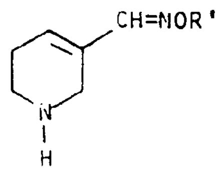

(II)

in which R′ retains the same meaning as previously, is subjected to the action of an alkoxycarbonylation agent capable of introducing the —$CO_2R$ radical, R being defined as previously, in order to obtain the corresponding compound of formula (I), which is subjected, if desired, to the action of an acid in order to form the salt.

17. Preparation process according to claim 16, characterized in that the alkoxycarbonylation agent is a product of formula (III) :

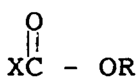

(III)

in which X represents a halogen atom, a paranitrophenoxy radical or an

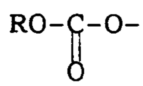

radical, R being defined as previously.

18. A variant of the process according to claim 16 or 17, characterized in that a compound of formula (IV):

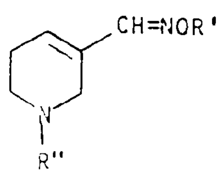

(IV)

in which R″ represents a saturated or unsaturated, linear, branched or cyclic alkyl radical containing up to 4 carbon atoms or a benzyl or phenethyl radical, is subjected to the action of an agent capable of cleaving the R″ group and of introducing the

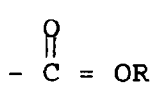

group, R being defined as previously, in order to obtain the corresponding compound of formula (I) :

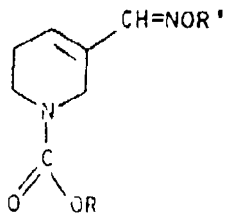

(I)

which is subjected, if desired, to the action of an acid so as to form the salt.

**Claims for the following Contracting States : ES, GR**

1. Process for preparing the compounds of formula (I) :

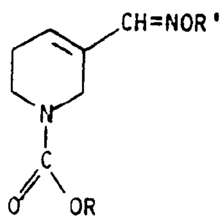

(I)

in which R′ represents a hydrogen atom, a saturated or unsaturated, linear, branched or cyclic alkyl radical containing up to 8 carbon atoms and R represents a saturated or unsaturated, linear, branched or cyclic alkyl radical containing up to 18 carbon atoms optionally substituted by the alkylsulphonyl radical containing up to 18 carbon atoms or the Si-$Alk_3$ radical in which Alk represents an alkyl radical containing up to 4 carbon atoms, a phenyl, benzyl or phenethyl radical optionally substituted by one or more substituents chosen from halogen atoms or the following radicals : methyl, ethyl, linear or branched propyl, linear or branched butyl, methoxy, ethoxy, linear or branched propoxy and linear or branched butoxy, as well as their addition salts with organic or mineral acids, characterized in that a compound of formula (II) :

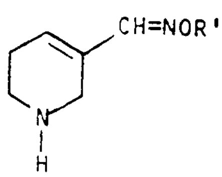

(II)

in which R′ retains the same meaning as previously, is subjected to the action of an alkoxycarbonylation agent capable of introducing the —$CO_2R$ radical, R being defined as previously, in order to obtain the corresponding compound of formula (I), which is subjected, if desired, to the action of an acid in order to form the salt.

2. Preparation process according to claim 1, characterized in that the alkoxycarbonylation agent is a product of formula (III) :

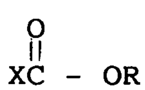

(III)

in which X represents a halogen atom, a paranitrophenoxy radical or an

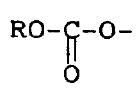

radical, R being defined as previously.

3. A variant of the process according to claim 1 or 2, characterized in that a compound of formula (IV) :

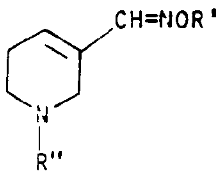

(IV)

in which R″ represents a saturated or unsaturated, linear, branched or cyclic alkyl radical containing up to 4 carbon atoms or a benzyl or phenethyl radical, is subjected to the action of an agent capable of cleaving the R″ group and of introducing the

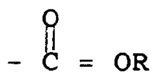

group, R being defined as previously, in order to obtain the corresponding compound of formula (I) :

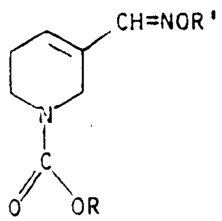

(I)

which is subjected, if desired, to the action of an acid so as to form the salt.

4. Process according to claim 1, 2 or 3 characterized in that a product capable of introducing the —$CO_2R$ radical in which R represents a saturated or unsaturated, linear, branched or cyclic alkyl radical containing up to 18 carbon atoms, a phenyl radical, a benzyl or phenethyl radical, is used at the start.

5. Process according to any one of claims 1 to 3, characterized in that a product of formula (II) or (IV) in which R′ represents a saturated or unsaturated, linear alkyl radical containing up to 4 carbon atoms, as well as their addition salts with organic or mineral acids, is used at the start.

6. Process according to any one of claims 1 to 3, characterized in that a product of formula (II) or (IV) in which R′ represents a methyl radical, as well as their addition salts with organic or mineral acids, is used at the start.

7. Process according to claim 4, characterized in that a product of formula (II) or (IV) in which R′ represents a methyl radical, as well as their addition salts with organic or mineral acids, is used at the start.

8. Process according to claim 4, characterized in that a product capable of introducing the —$CO_2R$ radical in which R represents a saturated or unsaturated, linear alkyl radical containing up to 4 carbon atoms as well as their addition salts with organic or mineral acids, is used at the start.

9. Process according to claim 4, characterized in that a product capable of introducing the —$CO_2R$ radical in which R represents an ethyl radical, as well as their addition salts with organic or mineral acids, is used at the start.

10. Process according to claim 4, characterized in that a product capable of introducing the —$CO_2R$ radical in which R represents an allyl radical, as well as their addition salts with organic or mineral acids, is used at the start.

11. Process according to claim 4, characterized in that a product capable of introducing the —$CO_2R$ radical in which R represents a phenyl radical, as well as their addition salts with organic or mineral acids, is used at the start.

12. Process according to any one of claims 1 to 3, characterized in that a product capable of introducing the —$CO_2R$ radical in which R represents a phenyl radical substituted by one or more of the radicals mentioned above, as well as their addition salts with organic or mineral acids, is used at the start.

13. Process according to any one of claims 1 to 3, characterized in that a product capable of introducing the —$CO_2R$ radical in which R represents a 4-chloro phenyl, 4-isopropyl phenyl or 3,4-dimethoxy phenyl radical, as well as their addition salts with organic or mineral acids, is used at the start.

14. Process according to claim 1 or 3 for the preparation of the methyl ether of of 4-chlorophenyloxycarbonyl-1,2,5,6-tetrahydropyridin-3-carboxaldehyde oxime or one of its addition salts with organic or mineral

acids, characterized in that a product of formula (II) or (IV) in which R′ represents a methyl radical and a product capable of introducing the $—CO_2R$ radical in which R represents a 4-chloro phenyl radical, are used at the start.